# EUROPÄISCHE PATENTANMELDUNG

(11) **EP 4 641 572 A1**
(43) Veröffentlichungstag der Anmeldung: **29.10.2025**
(21) Anmeldenummer: 24171812.1
(22) Anmeldetag: 23.04.2024
(51) Int. Cl.: G16B 5/30

(54) **VERFAHREN ZUR ANALYSE VON SCHALT- UND REGULATIONSPROZESSEN**

(71) Anmelder: Otto-von-Guericke-Universität Magdeburg, 39106 Magdeburg (DE)
(72) Erfinder: MARWAN, Wolfgang, Magdeburg (DE); HEINER, Monika, Cottbus (DE); HAAS, Markus, Magdeburg (DE)
(74) Vertreter: Braeuning Schubert Patentanwälte GbR

(57) **Zusammenfassung**

Der Gegenstand der vorliegenden Erfindung ist ein Verfahren zur Analyse von Schalt und Regulationsprozessen, welches folgende Schritte umfasst: a) Bereitstellung mindestens eines Reaktionssystems, b) Messen und auswählen mindestens eines Parameters an mindestens einem Reaktionssystem aus Schritt a), c) Mindestens eine Wiederholung von Schritt b), d) Erstellen mindestens einer Zeitserie mittels mindestens eines in Schritt b) ausgewählten Parameters, e) Diskretisieren der gemessenen Parameterwerte aus Schritt b) in der Zeitserie aus Schritt d) zur Erzeugung einer Abfolge von Mikrozuständen, f) Erzeugen eines gerichteten Graphen über Makrozustände aus der mindestens einen diskretisierten Zeitserie in Schritt e), g) Strukturelle Analyse des gerichteten Graphen aus Schritt f) zur kombinatorischen Ermittlung der Zahl der zeitlich korrelierten Änderungen der Mikrozustände in einer Teilmenge zufällig ausgewählter Systemkomponenten.

## Beschreibung

### Technisches Gebiet

Die vorliegende Erfindung betrifft ein Verfahren zur Analyse von Schalt- und Regulationsprozessen in mindestens einem Reaktionssystem.

### Stand der Technik

Die globale Dynamik komplexer Genregulationsnetzwerke wurde metaphorisch durch die Waddington-Landschaft veranschaulicht (Huang et al., 2009; Waddington, 1957). Eine biophysikalische Interpretation der Waddington-Landschaft als Quasi-Potential-Landschaft betrachtet das Genregulationsnetzwerk als ein dynamisches System, das die Wahrscheinlichkeiten von Genexpressionszuständen und von Übergängen bestimmt, die zwischen diesen Zuständen auftreten können (Graf and Enver, 2009; Huang, 2011; Macarthur et al., 2009; Moris *et al.,* 2016; Wu et al., 2017; Zhou and Huang, 2011). Die Waddington-Landschaft impliziert, dass die stabilen Zustände der Genexpression, die die verschiedenen Zelltypen hervorbringen, in einzelnen Zellen auf qualitativ alternativen Wegen erreicht werden können. Alternative Wege von einem Differenzierungszustand zu einem anderen wurden experimentell bei Physarum polycephalum für die Entwicklung von Amöben zu Plasmodien auf morphologischer Ebene (Solnica-Krezel et al., 1991) und bei klonalen Populationen von Säugerzellen auf molekularer Ebene nachgewiesen (Bargaje et al., 2017; Huang et al., 2007; Zhou *et al.,* 2016).

Die biologische Relevanz des Paradigmas von Waddington wird durch experimentelle Evidenz unterstützt (Huang et al., 2009; Wu et al., 2017) und wurde durch umfangreiche theoretische Studien unter Verwendung von Ansätzen der nichtlinearen Dynamik (Ferrell and Machleder, 1998; Ferrell Jr, 2012) oder der statistischen Physik (Bornholdt and Kauffman, 2019; Huang, 2011) erhärtet. Einzelzellsequenzierungsdaten tierischer Zellen wurden entsprechend im Licht der Waddington-Landschaft interpretiert, wobei Zellen aufgrund der Ähnlichkeit ihrer Genexpressionsmuster entlang rekonstruierter Entwicklungstrajektorien angeordnet werden (Saelens et al., 2019). Die Aussagekraft der so gewonnenen Pseudozeitreihen ist aus prinzipiellen Gründen begrenzt und ihre Interpretierbarkeit in Bezug auf die Waddington-Landschaft entsprechend fragwürdig (Sparta et al., 2023; Weinreb et al., 2018). Vor dem Hintergrund dieser Problematik wurden kürzlich Verfahren beschrieben, die die sequentielle Probenahme aus einzelnen Säugerzellen erlauben (Chen et al., 2022; Marcuccio, 2023), die die Relevanz und mögliche Anwendungsbreite des erfindungsgemäßen Verfahrens erweitern.

### Zusammenfassung der Erfindung

Aufgabe der vorliegenden Erfindung ist es daher, echte Zeitreihen der Zustände von interagierenden Komponenten in sich entwickelnden Reaktionssystemen zu messen und die konsekutiven Veränderungen der Zustände der Komponenten, die mit der Interaktion einhergehen, zu untersuchen.

Die Aufgabe der Erfindung wird gelöst durch die Bereitstellung eines Verfahrens zur Analyse von Schalt- und Regulationsprozessen, welches folgende Schritte umfasst:
a) Bereitstellen mindestens eines Reaktionssystems,
b) Messen und auswählen mindestens eines Parameters des mindestens einen Reaktionssystems aus Schritt a),
c) Mindestens eine Wiederholung von Schritt b),
d) Erstellen mindestens einer Zeitserie mittels mindestens eines in Schritt b) ausgewählten Parameters,
e) Diskretisieren der gemessenen Parameterwerte aus Schritt b) in der Zeitserie aus Schritt d) zur Erzeugung einer Abfolge von Mikrozuständen,
f) Erzeugen eines gerichteten Graphen über Makrozustände aus der mindestens einen diskretisierten Zeitserie in Schritt e),
g) Strukturelle Analyse des gerichteten Graphen aus Schritt f) zur kombinatorischen Ermittlung der Zahl der zeitlich korrelierten Änderungen der Mikrozustände in einer Teilmenge zufällig ausgewählter Systemkomponenten.

Ein Reaktionssystem beschreibt eine oder mehrere Zellen, eine Zellpopulation, ein *in vitro* System, ein Teilsystem in einer Zelle oder ein extrazelluläres System.

Parameter beziehen sich auf verschiedene messbare Größen oder Eigenschaften, die verwendet werden, um die Struktur, Dynamik oder Funktion von Reaktionssystemen innerhalb eines Netzwerkes zu charakterisieren. Ein Parameter definiert einen Mikrozustand.

Die Messung in Schritt b) zum Erhalt eines Messwertes kann sowohl an einer Probe, welche dem mindestens einen Reaktionssystem entnommen wurde, als auch physikalisch, beispielsweise in Form von Fluoreszenz erfolgen.

Ein Mikrozustand bezieht sich auf den detaillierten Zustand eines Parameters in dem mindestens einen Reaktionssystem auf molekularer Ebene. Somit kann ein Mikrozustand die Konzentration eines Biomoleküls oder die spezifische Anordnung von Biomolekülen relativ zueinander sein. Ein Makrozustand ist die Kombination von Mikrozuständen, welcher den aggregierten oder globalen Zustand des mindestens einen Reaktionssystems auf einer höheren Ebene beschreibt. Systemkomponenten in Schritt f) sind definiert als Biomoleküle, z.B. mRNAs, Proteine, Metabolite einschließlich deren kovalenten Modifikationen oder als Komplexe (Aggregate) von Biomolekülen.

In einer bevorzugten Ausführungsform der vorliegenden Erfindung, wird ein Verfahren bereitgestellt, bei dem das mindestens eine Reaktionssystem in eukaryotischen oder prokaryotischen Zellen enthalten ist oder aus solchen Zellen besteht.

Bevorzugt wird weiterhin eine Ausführungsform der vorliegenden Erfindung, bei dem ein Verfahren bereitgestellt wird, in dem nach Schritt a) ein Schritt a1) erfolgt, in dem an dem mindestens einen Reaktionssystem eine Pertubation erfolgt.

Pertubation bedeutet, dass eine Änderung der Aktivität einer Komponente in einem Reaktionssystem hervorgerufen wird. Diese Änderung erfolgt durch Stimulation, pharmakologische Substanzen oder anderer Agentien wie Antikörper oder Aptamere, *etc.,* oder beispielsweise mittels optogenetischer Methoden, genetischer oder gentechnischer Manipulation, oder durch Fusion von Zellen mit unterschiedlicher genetischer oder physiologischer Konstitution, oder durch Mischung entsprechender Reaktionssysteme.

Weiterhin wird ein Verfahren gemäß der vorliegenden Erfindung bevorzugt, bei dem die Pertubation als Induktion einer Zelle durch Stimulation erfolgt. Die Stimulation erfolgt durch einen Lichtreiz.

Bevorzugt wird weiterhin ein Verfahren gemäß der vorliegenden Erfindung, bei dem in Schritt d) ein Startpunkt aus den Messdaten in Schritt b) für die Erstellung der Zeitserie bestimmt wird.

Insbesondere bevorzugt ist ein Verfahren gemäß der vorliegenden Erfindung, bei dem in Schritt b) der mindestens eine Parameter die zelluläre Konzentration von Biomolekülen, von deren kovalenten Modifikationen oder die Konzentration vorhandener Komplexe (Aggregate) von Biomolekülen ist. Biomoleküle sind z.B. mRNAs, Proteine oder Metabolite unter Einbeziehung deren kovalenten Modifikationen sowie die relative Anordnung solcher Biomoleküle unter Bildung von Komplexen (Aggregaten).

Zusätzlich bevorzugt ist ein Verfahren gemäß der vorliegenden Erfindung, bei dem ein Logarithmus der Konzentration jedes Parameters gegen ein definiertes zeitliches Intervall aufgetragen und graphisch dargestellt wird.

Weiterhin bevorzugt ist ein Verfahren gemäß der vorliegenden Erfindung, bei dem die mindestens eine Wiederholung in Schritt c) in einem zeitlich definierten Intervall erfolgt.

Des Weiteren ist ein Verfahren gemäß der vorliegenden Erfindung bevorzugt, bei dem die Makrozustände durch die Änderung mindestens eines Parameters der Mikrozustände in Schritt e) hervorgerufen werden.

Bevorzugt ist außerdem ein Verfahren gemäß der vorliegenden Erfindung, bei dem die Änderung ein Anstieg oder ein Abfall, oder eine Oszillation eines Parameters oder keine Veränderung ist.

Besonders bevorzugt ist ein Verfahren gemäß der vorliegenden Erfindung, bei dem die Abfolge von Makrozuständen in Form eines gerichteten Graphen dargestellt wird, wobei der gerichtete Graph ein endlicher Automat ist. Ein endlicher Automat ist ein Modell eines Verhaltens, bestehend aus Makrozuständen, Zustandsübergängen und Aktionen.

Insbesondere bevorzugt ist ein Verfahren gemäß der vorliegenden Erfindung, bei dem der endliche Automat als ein Petrinetz dargestellt ist. Ein Petrinetz ist eine mathematische Struktur, die Reaktionsnetzwerke formal, auch graphisch darzustellen, vermag. Es ist ein gerichteter bipartiter Graph, bestehend aus Plätzen und Transitionen, die mit Kanten verbunden sind. Plätze können Komponenten, wie Makrozustände eines Reaktionssystems darstellen, und die Transitionen repräsentieren die Übergänge zwischen Makrozuständen. In der graphischen Darstellung werden Plätze als Kreise und Transitionen als Rechtecke oder Quadrate repräsentiert.

Weiterhin ist ein Verfahren gemäß der vorliegenden Erfindung bevorzugt, bei dem in Schritt g) unterschiedliche Makrozustände bestimmt werden, die in einem gemeinsamen Makrozustand enden.

Des Weiteren ist ein Verfahren gemäß der vorliegenden Erfindung bevorzugt, bei dem in Schritt g) die Anzahl gleicher Makrozustandsänderungen bestimmt wird, die in einem gemeinsamen Makrozustand enden.

Bevorzugt ist außerdem ein Verfahren gemäß der vorliegenden Erfindung, bei dem eine Änderung im Mikrozustand eines Parameters identifiziert wird, der vor dem Hintergrund anderer Parameter auftritt, deren Mikrozustand sich zu dem jeweiligen Zeitpunkt noch nicht oder nicht mehr ändert.

Des Weiteren ist ein Verfahren gemäß der vorliegenden Erfindung bevorzugt, bei dem in Schritt g) alle Anfangs- und Endmakrozustände und deren Anzahl bestimmt wird.

### Kurze Beschreibung der Figuren

Die vorliegende Erfindung wird mit den beigefügten Zeichnungen näher erläutert. Es zeigt:
- Fig. 1A: die beispielhafte graphische Darstellung diskretisierter Genexpressionszustände gemäß dem erfindungsgemäßen Verfahren;
- Fig. 1B: eine exemplarische graphische Darstellung des Ablaufs einer Diskretisierung gemäß Figur 1 A;
- Fig. 1C: ein Beispiel für eine Genexpressionszustandstabelle gemäß dem erfindungsgemäßen Verfahren;
- Fig. 1D: ein Petrinetz, welches gemäß dem erfindungsgemäßen Verfahren mittels der Genexpressionszustandstabelle aus Figur 1C konstruiert wurde;
- Fig. 1E: ein zweites Beispiel für ein mittels des erfindungsgemäßen Verfahrens konstruiertes Petrinetz;
- Fig. 2A: ein mittels des erfindungsgemäßen Verfahrens konstruiertes Petrinetz für das Gen anxA;
- Fig. 2B: ein mittels des erfindungsgemäßen Verfahrens konstruiertes Petrinetz für das Gen bzpJ;
- Fig. 3: eine graphische Darstellung eines Hubs (A) und einer Raute (B) als strukturelle Elemente einer Petrinetz-Struktur;
- Fig. 4: graphische Darstellungen möglicher Anordnungen von Hubs und Rauten als direkt verbundene Teile einer Petrinetz-Struktur;
- Fig. 5: einen Auszug aus einer Liste von Hubs;
- Fig. 6: die zeitliche Häufigkeitsverteilung von Hubs in Abhängigkeit von einer Mindestanzahl von Zellen, die zu einem Hub beigetragen haben

### Ausführliche Beschreibung der Erfindung

Das erfindungsgemäße Verfahren kann beispielsweise zur Rekonstruktion von genregulatorischen Netzwerken zur Analyse von Schalt- und Regulationsprozesse verwendet werden, insbesondere um
(1) nebenläufige von sequentiellen Prozessen zu unterscheiden,
(2) den Grad der Nebenläufigkeit von Prozessen zu quantifizieren,
(3) die zeitliche Reihenfolge von Schaltvorgängen zu ermitteln,
(4) die Stärke der Korrelation (Koppelung) von Schaltvorgängen zu messen und
(5) die Wahrscheinlichkeit, mit der bestimmte Zwischen- oder Endzustände erreicht werden und damit z.B. die Stärke von Attraktoren eines dynamischen Systems auf der Ebene von Biomolekülen im oben bezeichneten Sinn zu bestimmen.

Die Wechselwirkungen von Genen und Proteinen, stellen einen essentiellen Teil einer Maschinerie (genregulatorisches Netzwerk) dar, wie sie beispielsweise der Entwicklung des menschlichen Körpers aus einer befruchteten Eizelle unter Hervorbringen aller Körperfunktionen zugrunde liegt.

Sogenannte Gen-Expressionsraten, welche ein Genexpressionsmuster bilden, liefern eine Beschreibung bzw. Repräsentation eines genregulatorischen Netzwerks bzw. eines aktuellen Zustands des genregulatorischen Netzwerks. Somit repräsentiert das Genexpressionsmuster einer Zelle den aktuellen Zustand des genregulatorischen Netzwerks dieser Zelle.

In den Booleschen Modellen genregulatorischer Netzwerke kann jedes Gen zwei Zustände einnehmen: "an" oder "aus", je nachdem, ob es transkriptionell aktiv ist oder nicht. Wenn ein Gen angeschaltet ist, steigt die Konzentration seiner mRNA an, bis das Gen wieder abgeschaltet wird oder bis Synthese und Abbau der mRNA gleich schnell erfolgen und die Konzentration der mRNA ein konstant hohes Niveau erreicht, nachdem sich ein Fließgleichgewicht eingestellt hat. Mögliche Faktoren, die die mRNA-Konzentration beeinflussen, sind neben der Transkription auch die Regulation der Stabilität und damit des Abbaus einer mRNA. Die Konzentration einer mRNA nimmt zu, solange die Biosyntheserate höher ist als die Abbaurate, und sie nimmt ab, wenn die Abbaurate höher ist als die Biosyntheserate. Aus dem zeitlichen Verlauf der mRNA-Konzentration kann somit auf die differentielle Regulation eines Gens geschlossen werden, auch wenn eine Unterscheidung zwischen Regulation der Transkription und Regulation der mRNA-Stabilität nicht ohne weitere Kriterien möglich ist. Trägt man den Logarithmus der mRNA-Konzentration gegen die Zeit auf, so ist die Steigung der kinetischen Kurve proportional der x-fachen Änderung der mRNA-Konzentration pro Zeitintervall. Bei der visuellen Auswertung vieler halblogarithmischer Kinetiken kann beobachtet werden, dass größere (signifikante) Änderungen der mRNA-Konzentration häufig mit Diskontinuitäten einhergehen, die sich in scharfen Knicken in den kinetischen Kurven äußern. Zwischen den Knicken ist der Kurvenverlauf im semilogarithmischen Plot oft weitgehend linear, was auf einen exponentiellen Abfall der mRNA-Konzentration im "off'-Zustand und einen exponentiellen Anstieg mit autokatalytischer Kinetik der mRNA-Bildung im "on"-Zustand hindeutet. Mittels des erfindungsgemäßen Verfahrens ist es durch die Definition von fünf Expressionszuständen möglich, die Änderung des Expressionszustands eines Gens gerade auch in Korrelation mit anderen Genen noch genauer zu analysieren als es mit den Booleschen Modellen möglich ist.

Bei konstanten Expressionsniveaus wurde zusätzlich zwischen hoch, mittel und niedrig unterschieden. In den Zuständen hoch und mittel ist das Gen transkriptionell aktiv (also im Prinzip angeschaltet), aber die Synthese- und Abbauraten halten sich die Waage.

### Beschreibung der Ausführungsbeispiele

Die nachfolgenden Beispiele erläutern das erfindungsgemäße Verfahren näher, ohne den Umfang der Erfindung zu beschränken.

In allen Beispielen wurden vielkernige Riesenzellen (Plasmodien) von P. *polycephalum* verwendet, deren Protoplasma sich aufgrund der starken Strömung ständig durchmischt und so ein homogenes protoplasmatisches Reaktionsvolumen bildet.

Jede Zeitreihe wurde durch wiederholte Probenahme einer einzelnen Plasmodiumzelle erhalten, wobei eine Probe vor und die anderen 10 Proben nach einem dunkelroten Lichtimpuls entnommen wurden, der die Sporulation in jeder der analysierten Plasmodiumzellen auslöste. Von jeder Plasmodiumzelle wurden zu jedem Zeitpunkt zwei Protoplasmaproben entnommen und aus jeder Probe wurde die Gesamt-RNA isoliert. Die Konzentration jeder analysierten mRNA wurde zweimal in jeder RNA-Präparation auf einer dem Fachmann bekannten Weise mittels GeXP RT-PCR bestimmt, um zwei technische Replikate von jedem der beiden biologischen Replikate zu erhalten, was vier Expressionswerten für jede analysierte mRNA zu jedem Zeitpunkt in jeder Plasmodiumzelle entspricht. Insgesamt wurde eine Transkriptmenge von 122 Genen erhalten (Tabelle A, Anhang).

### Beispiel 1

Die normalisierten, gemittelten Expressionswerte jedes analysierten Gens wurden für jede Zelle getrennt gegen die Zeit aufgetragen. Zur Darstellung der x-fachen Veränderungen in der Genexpression, wird der Logarithmus der Konzentration jeder mRNA gegen die Zeit in einem semilogarithmischen Diagramm aufgetragen.

Um einen qualitativen Vergleich der Expressionskinetik eines Gens zwischen verschiedenen Zellen zu ermöglichen, werden die zeitlichen Veränderungen der mRNA-Konzentration diskretisiert, um Zustände abzuleiten.

Um die zeitabhängigen Änderungen der Konzentration einer genspezifischen mRNA innerhalb einer definierten Zeit in einer Zeitreihe zu diskretisieren, werden fünf Zustände der Genexpression definiert, je nachdem ob die mRNA-Konzentration über einen bestimmten Zeitraum ansteigt (on), abfällt (off) oder konstant bleibt. Ein konstantes Niveau kann niedrig (lo), mittel (in) oder hoch (hi) sein.

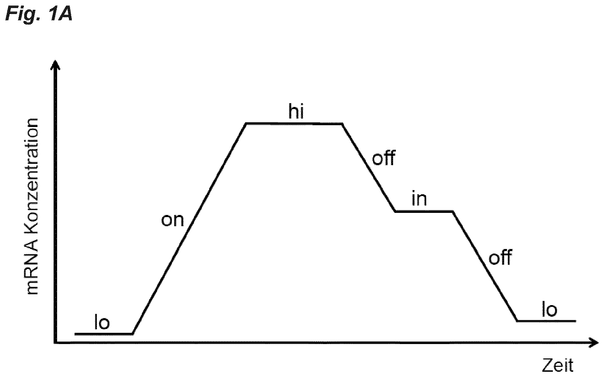

In Figur 1A ist der Zeitverlauf der logarithmierten Abundanz einer genspezifischen mRNA schematisch dargestellt, welche zu den Genexpressionszuständen gemäß dem erfindungsgemäßen Verfahren diskretisiert wurde. Aus der graphischen Darstellung ist zu entnehmen, dass sich die Aktivität des Gens und damit der Genexpressionszustand über die Zeit verändert.

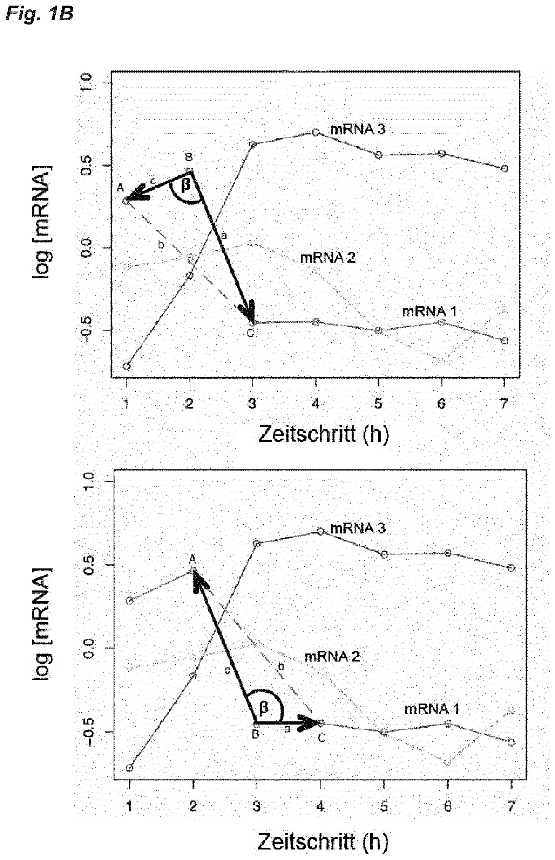

Technisch erfolgt das Diskretisieren gemäß dem erfindungsgemäßen Verfahren durch Auswertung zeitlich aufeinanderfolgender Datenpunkte jedes halblogarithmischen Diagramms, indem der Winkel berechnet wird, der von jedem Doppelpfeil eingeschlossen wird, der jeweils drei zeitlich aufeinanderfolgende Datenpunkte verbindet. Figur 1B zeigt eine exemplarische graphische Darstellung des Ablaufs des Diskretisierens gemäß dem erfindungsgemäßen Verfahren. Das Diskretisieren erfolgt durch Scannen aufeinander folgender Zeitpunkte einer Einzelzell-Zeitreihe der Konzentration einer bestimmten mRNA (log[mRNA] gegen die Zeit) mit Hilfe eines Doppelpfeil-Algorithmus, um plötzliche Änderungen in der Expressionskinetik zu erkennen. Der Algorithmus wertet den Winkel aus, der von zwei Pfeilen eingeschlossen wird, von denen der eine in die Vergangenheit und der andere in die Zukunft zeigt, bezogen auf den aktuellen Zeitpunkt. Plötzliche Veränderungen (Diskontinuitäten bzw. Knicke) im Verlauf der Kurve bewirken einen kleinen Winkel β zwischen den beiden Pfeilen. Die Ausgabe des Algorithmus wird zusammen mit der x-fachen Änderung der mRNA-Konzentration zwischen aufeinander folgenden Datenpunkten in diskrete Genexpressionszustände wie in Figur 1A dargestellt, übersetzt.

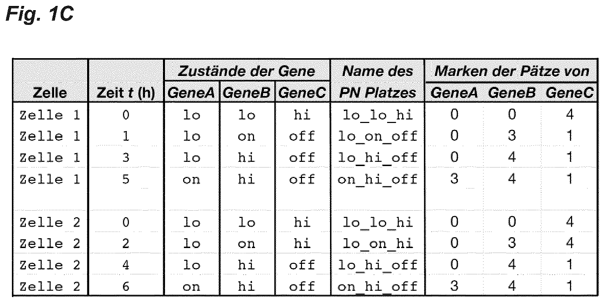

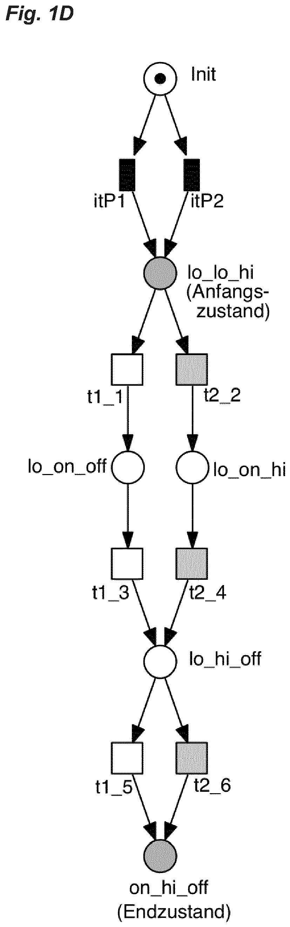

Für die automatische Konstruktion der Petrinetze werden die Ergebnisse des Doppelpfeilalgorithmus in eine Tabelle eingetragen, in der die Zustände aufgelistet sind, die aus dem zeitlichen Verlauf der Transkriptkonzentration jedes Gens abgeleitet werden. In der Figur 1C ist eine derartige Zustandstabelle beispielhaft dargestellt, welche bei Durchführung des erfindungsgemäßen Verfahrens entsteht. In der Tabelle werden die Zustände für drei Gene und zwei Zellen dargestellt. Die Zustandstabelle wird verwendet, um einen entsprechenden Zustandsautomaten in Form eines Petrinetzes zu konstruieren, welches in Figur 1D dargestellt ist, dass das Verhalten aller betrachteten Zellen bzgl. der ausgewählten Gene repräsentiert.

Für die grafische Darstellung wurden die Petrinetze im ANDL-Format kodiert (Abstract Net Description Language; (Heiner et al., 2013)) und mit einem R-Script als Text exportiert. Jedes Petrinetz wurde in zwei Versionen erzeugt, eine Version, in der die Übergänge jeder Zelle als einzelne, möglicherweise parallele Transitionen dargestellt wurden, und eine Version, in der die parallelen Transitionen, die die Plätze verbinden, zu einer gemeinsamen Transition zusammengefasst wurden. Anschließend wurde jede ANDL-Datei in Snoopy importiert, um das graphische Layout eines ausführbaren Petrinetzes zu erhalten.

Um Ähnlichkeiten und Unterschiede zwischen den einzelnen Zellen darzustellen, wird jeder Übergang zwischen aufeinanderfolgenden Zuständen einer Zelle durch eine zellspezifische Transition repräsentiert, die nach der Zell-ID-Nummer und dem Zeitpunkt des Übergangs benannt wird. Zur graphischen Darstellung und Veranschaulichung werden die Übergänge entweder entsprechend der Zell-ID-Nummer, wie in Figur 1D dargestellt und/oder entsprechend der Zeit farbkodiert, zu der der Übergang zwischen den Zuständen stattfand.

Die Transitionen, die die Übergänge derselben Zelle darstellen, können in derselben Farbe hervorgehoben werden. In dem vorliegenden Beispiel beginnen beide Zellen im gleichen Ausgangszustand (grau gefärbter Kreis mit der Bezeichnung lo_lo_hi, Initial State), nehmen dann aber unterschiedliche Wege (d. h. sie durchlaufen verschiedene Zwischenzustände) und befinden sich schließlich im gleichen Endzustand (grau gefärbter Kreis mit der Bezeichnung lo_hi off), der ihnen am Ende des Experiments zugewiesen wird.

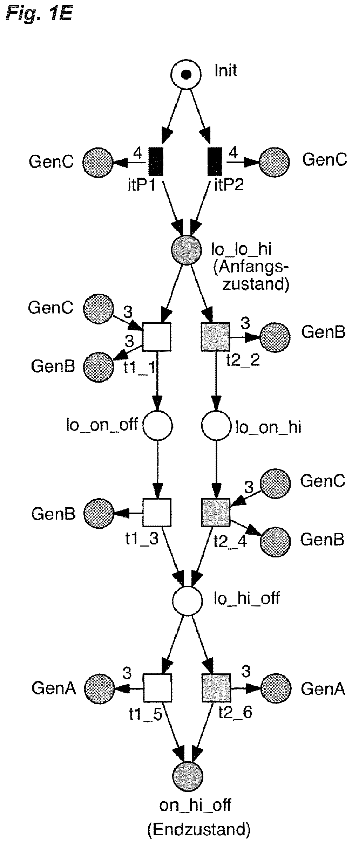

Um eine erste Markierung des Petrinetzes zu erhalten und den konkreten Anfangszustand eines Netzwerkes zu definieren, werden alle Plätze, die Anfangszustände repräsentieren, mit der Init-Stelle als Vorplatz mit einer so genannten immediaten Transition (einer sofort schaltenden Transition, dargestellt als schwarzes Rechteck) verbunden, die eine Marke in einen derjenigen Plätze bewegt, die die möglichen Anfangszustände des Reaktionsnetzwerkes repräsentieren. Alle Plätze, die weder Anfangs- noch Endzustände repräsentieren, haben immer genau so viele eingehende wie ausgehende Kanten. Um eine Aktivität der einzelnen Gene direkt ablesen und Simulationskurven erstellen zu können, kann ein Petrinetz wahlweise mit zusätzlichen Plätzen versehen werden, deren Markierung die Aktivität der einzelnen Gene repräsentiert, wie es in Figur 1E dargestellt ist.

### Beispiel 2

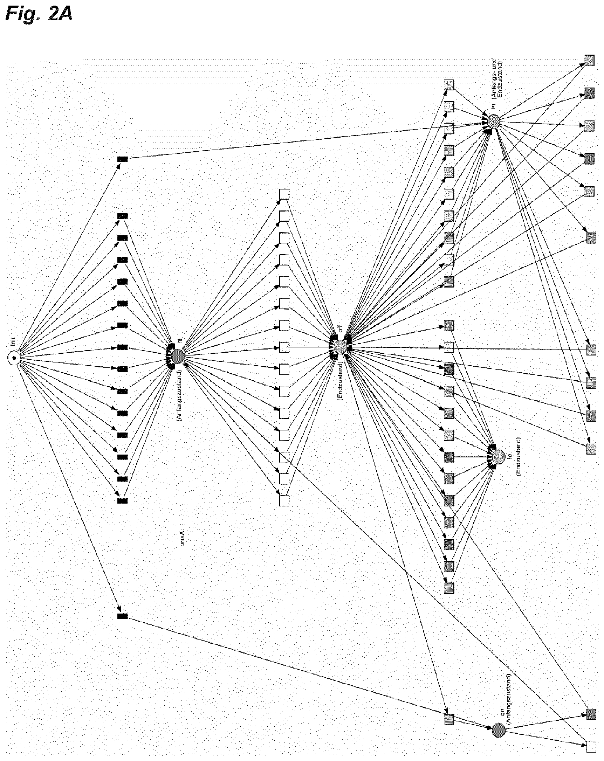

In Figur 2A wird ein Petrinetz gezeigt, um das Gen anxA mittels des erfindungsgemäßen Verfahrens zu analysieren. Für das Experiment wurden insgesamt Proben aus 16 Plasmodiumzellen entnommen.

Bei den meisten Zellen (14/16) war die Expression der anxA-mRNA zu Beginn des Experiments hoch und fiel im Verlauf einer Stunde nach dem FR-Lichtreiz ab. Danach blieb die mRNA-Konzentration bei zehn Zellen vorübergehend konstant, bevor das Gen wieder abgeschaltet wurde und die mRNA-Konzentration entsprechend sank. Vier Zellen gingen direkt von "off" in den Endzustand "lo" ohne den Umweg über "in" zu nehmen. Die unterschiedlichen Grauschattierungen der Übergänge zeigt, dass das Umschalten zwischen den Zuständen bei den meisten Zellen in derselben zeitlichen Reihenfolge, aber zu individuell unterschiedlichen Zeitpunkten stattfand.

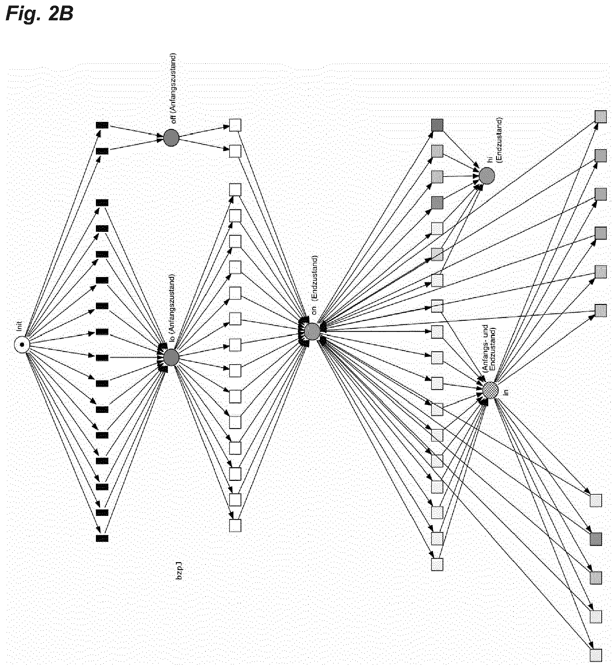

Ein ähnliches Verhalten hinsichtlich zwischengeschalteter "in" Zustände zeigt sich für andere Gene, so zum Beispiel das bzpJ-Gen. Das entsprechende Petrinetz ist in Figur 2 B dargestellt.

Mittels des erfindungsgemäßen Verfahrens ist es möglich, zeitliche Regulationsmuster einzelner analysierter Gene sowie die Variabilität der Regulationsmuster zu betrachten. Sowohl das Ein-Gen-Petrinetz für das anxA-Gen in Figur 2A, als auch für das bzpJ-Gen in Figur 2B zeigen im Wesentlichen nur eine oder wenige Haupttrajektorien von Genexpressionszuständen, denen die Zellen folgten. Während sich diese Haupttrajektorien auf die zeitliche Abfolge des Wechsels zwischen den Expressionszuständen eines bestimmten einzelnen Gens bezogen, gab es erhebliche Unterschiede in den chronologischen Zeitpunkten, zu denen diese Zustandsübergänge in einzelnen Zellen auftraten.

### Beispiel 3

Mittels des erfindungsgemäßen Verfahrens können mit Hilfe von Hubs und Rauten im Petrinetz Gene identifiziert werden, die ihren Expressionsstatus in korrelierter Weise ändern. Rauten zeigen darüber hinaus Änderungen im Expressionszustand von Genen, die vor dem Hintergrund anderer Gene auftreten, deren Expressionszustand sich zu dem jeweiligen Zeitpunkt noch nicht oder nicht mehr ändert, und enthüllen damit die zeitliche Teilordnung der differentiellen Regulation.

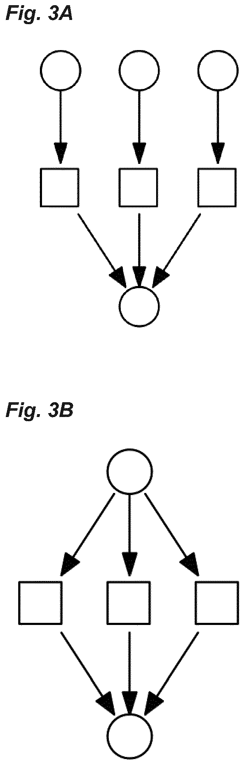

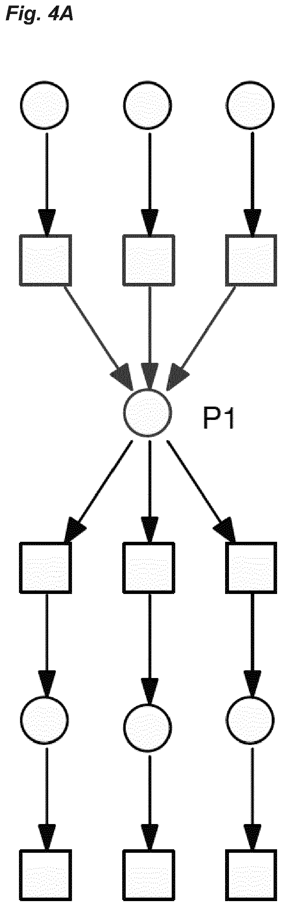

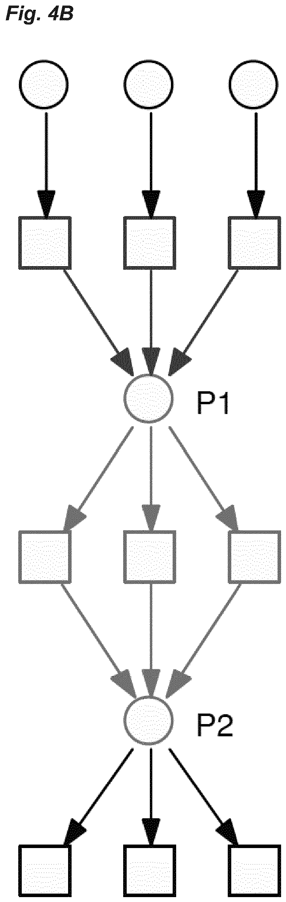

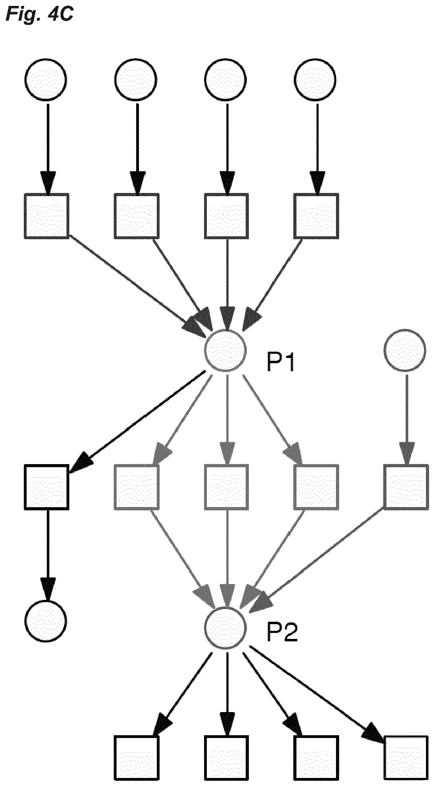

In Figur 3 werden mögliche strukturelle Darstellungen von Hubs (A) und Rauten (B) innerhalb einer Petrinetz-Struktur gezeigt. Zudem können Hubs und Rauten Strukturmotive eines Petrinetzes sein und innerhalb eines Petrinetzes auch direkt miteinander verbunden sein, wie es beispielhaft in Figur 4 gezeigt ist.

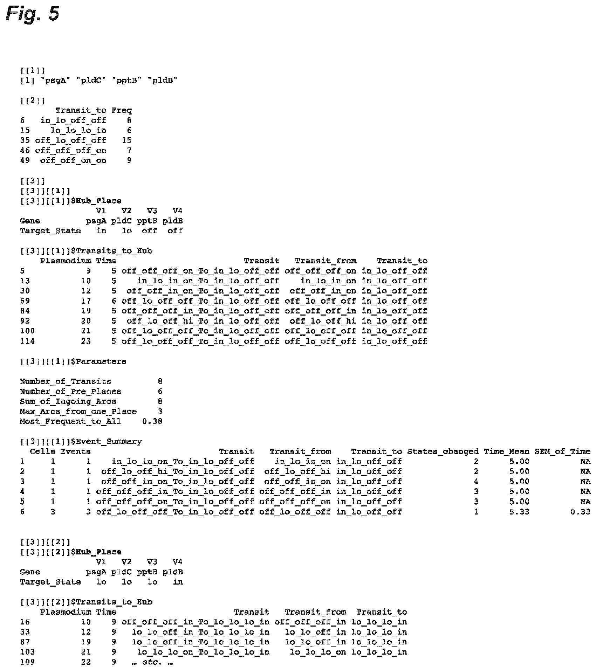

Das erfindungsgemäße Verfahren wurde für alle Kombinationen von 72 stark differentiell regulierten Genen in den Plasmodiumzellen verwendet, um in den erstellten Petrinetzen nach Hubs zu suchen, die durch eine bestimmte Mindestanzahl eingehender Kanten definiert sind. Jeder Hub wurde charakterisiert durch die Kombination der Namen der Gene, für die das Petrinetz konstruiert wurde, den Expressionsstatus dieser Gene, wie er durch den Hub-Platz repräsentiert wird, die ID-Nummer der Zellen, in denen die Änderung des Expressionsstatus, wie er durch den Hub-Platz repräsentiert wird, aufgetreten ist, die Zeiten, zu denen die Übergänge in jeder Zelle aufgetreten sind, und die Anzahl und Identität der Vor-Plätze der Übergänge, d.h. der Plätze, die direkt in den Hub führen. Figur 5 zeigt einen beispielhaften Auszug aus einer Liste der Hubs.

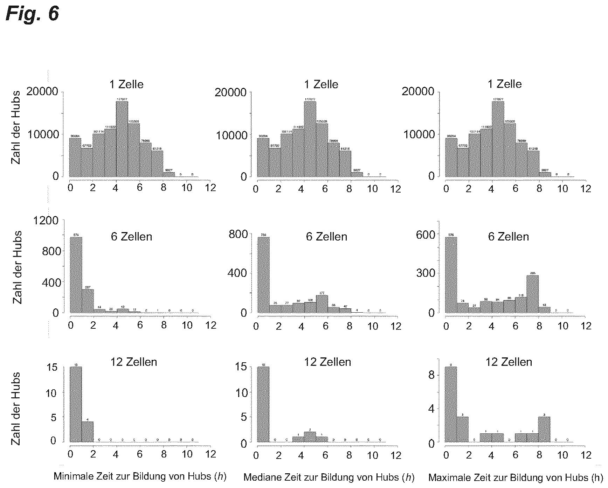

Somit ist es mittels des erfindungsgemäßen Verfahrens möglich, eine quantitative Untersuchung der Gene durchzuführen, indem deren Expressionszustände über die Zeit verglichen werden. Diese Vergleiche lassen eine Einschätzung dazu zu, ob eine Änderung der Expressionszustände mehrerer Gene auf koordinierte Änderungen oder auf zufällige Änderungen zurückzuführen sind. Wie in Figur 6 gezeigt, werden die Unterschiede zwischen zufälligen und koordinierten Veränderungen deutlich, wenn die zeitliche Verteilung der Hubs in Abhängigkeit von der Mindestanzahl der Zellen betrachtet wird, die zu einem Hub beigetragen haben. Die Anzahl der Hubs wurde gegen die minimale, mediane und maximale Zeit ihres Auftretens aufgetragen, in Abhängigkeit von der minimalen Anzahl der Zellen, die einen Hub bildeten. Die Verteilungen für eine Mindestanzahl von einer oder zwei Zellen waren eher zufällig, mit einem Gipfel im Bereich des "Point of no return" (4-5h), an dem sich wahrscheinlich die Expression vieler Gene änderte. Bei einer Mindestanzahl von sechs oder mehr Zellen waren die Verteilungen qualitativ unterschiedlich im Vergleich zu den Verteilungen für eine Mindestanzahl von nur einer Zelle. Es gab eine hohe Anzahl von Hubs, die während der ersten Stunde auftraten, d.h. unmittelbar nach dem dunkelroten induktiven Lichtimpuls. Betrachtet man die mediane oder die maximale Zeit der Übergänge eines Hubs, so gibt es eine beträchtliche Anzahl von Änderungen, die während der gesamten Beobachtungszeit, mindestens bis zu 7-8 Stunden nach dem Lichtpuls auftraten, was darauf hindeutet, dass korrelierte Änderungen der Genexpression während dieses gesamten Zeitraums stattfanden. Wenn alle Gene, für die mehrere Kombinationen gefunden wurden, in ihrer Expression strikt korreliert wären, müssten alle Transitionen, die zu den entsprechenden Plätzen eines solchen Hubs führen, von der gleichen Gruppe von Zellen stammen. Somit können auch Genpaare mittels des erfindungsgemäßen Verfahrens identifiziert werden, die häufiger zum Auftreten eines Hubs beitragen als andere, was dann auf eine potentielle Ko-Regulierung hindeutet.

Nachfolgend wird das erfindungsgemäße Verfahren noch einmal in anderen Worten zusammengefasst:
Verfahren zur Analyse von Schalt- und/oder Regulationsprozesse, welches folgende Schritte umfasst:
a) Erstellen einer oder mehrerer Zeitserien, jeweils durch Bestimmung eines oder mehrerer Parameter, die zeitlich aufeinanderfolgende Zustände des betrachteten Systems repräsentieren. Der Beginn der Zeitserien kann durch eine Perturbation, beispielsweise die Induktion einer Zelle oder eines biochemischen Reaktionssystems, oder durch ein anderes Vorkommnis definiert sein.
b) Diskretisierung, das heißt Umwandlung der Zeitserie in eine Abfolge definierter (diskreter) Zustände. Ein Zustand kann, aber muss nicht, auch durch die Art und Weise definiert sein, in der sich ein Parameter ändert. Die Änderung könnte also beispielsweise ein Anstieg, ein Abfall oder eine Oszillation innerhalb des Zeitfensters sein, dem der Zustand zugeordnet wird.
c) Kombinatorische Suche nach zeitlich korrelierten Änderungen des Zustands in einer Teilmenge (Subset) zufällig ausgewählter Systemkomponenten (und/oder Individuen bzw. Systeme) und Ermittlung der Häufigkeit, mit der diese Zustandsänderungen in korrelierter Weise erfolgen. Diese Suche kann und sollte systematisch erfolgen, so daß alle für eine Teilmenge (Subset) möglichen Kombinationen der Komponenten der Teilmenge ausgewertet werden. In der praktischen Durchführung kann diese Suche durch die Konstruktion eines endlichen Automaten (oder einer entsprechenden mathematischen Struktur), beispielsweise in Form eines Petri-Netzes, und die strukturelle Analyse des Automaten bzw. der mathematischen Struktur erfolgen.

In diesem Dokument sind folgende Schriften zitiert:
Bargaje, R., Trachana, K., Shelton, M. N., McGinnis, C. S., Zhou, J. X., Chadick, C., Cook, S., Cavanaugh, C., Huang, S., Hood, L., 2017. Cell population structure prior to bifurcation predicts efficiency of directed differentiation in human induced pluripotent cells. Proc. Nat. Acad. Sci. USA 114, 2271-2276.
Bornholdt, S., Kauffman, S., 2019. Ensembles, dynamics, and cell types: Revisiting the statistical mechanics perspective on cellular regulation. Journal of Theoretical Biology 467, 15-22.
Ferrell, J. E. J., Machleder, E. M., 1998. The biochemical basis of an all-or-none cell fate switch in Xenopus oocytes. Science 280, 895-898.
Ferrell Jr, J. E., 2012. Bistability, bifurcations, and Waddington's epigenetic landscape. Current Biology 22, R458-R466.
Heiner, M., Schwarick, M., Wegener,J., 2015. Charlie - An Extensible Petri Net Analysis Tool. In:
   Devillers R., Valmari A. (eds) Application and Theory of Petri Nets and Concurrency. PETRI NETS 2015. Lecture Notes in Computer Science, vol 9115. Springer, Cham. pp. 200-211.
   Hopfensitz, M., Müssel, C., Maucher, M., Kestler, H. A., 2012. Attractors in Boolean networks: a tutorial. Computational Statistics 28, 19-36.
   Huang, S., 2011. The molecular and mathematical basis of Waddington's epigenetic landscape: A framework for post-Darwinian biology? BioEssays 34, 149-157.
   Huang, S., Ernberg, 1., Kauffman, S., 2009. Cancer attractors: a systems view of tumors from a gene network dynamics and developmental perspective. Semin Cell Dev Biol 20, 869-876.
   Huang, S., Guo, Y.-P., May, G., Enver, T., 2007. Bifurcation dynamics in lineagecommitment in bipotent progenitor cells. Developmental Biology 305, 695-713.
   Macarthur, B. D., Ma'ayan, A., Lemischka, I. R., 2009. Systems biology of stem cell fate and cellular reprogramming. Nat Rev MolCell Biol, 1-10.
   Machado, D., Costa, R. S., Rocha, M., Ferreira, E. C., Tidor, B., Rocha, I., 2011. Modeling formalisms in Systems Biology. AMB Express1, 45.
   Marquardt, P., Werthmann, B., Raetzel, V., Haas, M., Marwan, W., 2021. Quantifying 35 transcripts in a single tube: model-based calibration of the GeXP multiplex RT-PCR assay. BMC Biotechnology 21, 29.
   Moris, N., Pina, C., Arias, A. M., 2016. Transition states and cell fate decisions in epigenetic landscapes. Nature Reviews Genetics 17, 693-703.
   Pretschner, A., Pabel, S., Haas, M., Heiner, M., Marwan, W., 2021. Regulatory dynamics ofc ell differentiation revealed by true time series from multinucleate single cells. Front. Genet. 11, 612256.
   Rätzel, V., Marwan, W., 2015. Gene expression kinetics in individual plasmodial cells reveal alternative programs of differential regulation during commitment and differentiation. Develop. Growth Differ. 57, 408-420.
   Saelens, W., Cannoodt, R., Todorov, H., Saeys, Y., 2019. A comparisonof single-cell trajectory inference methods. Nature Biotechnology 37, 547-554.
   Sauer, H. W., Babcock, K. L., Rusch, H. P., 1969a. Sporulation in Physarum polycephalum. A model system for studies on differentiation. Exp. Cell Res. 57, 319-327.
   Solnica-Krezel, L., Burland, T. G., Dove, W. F., 1991. Variable pathways for developmental changes of mitosis and cytokinesis in Physarum polycephalum. The Journal of Cell Biology 113, 591-604.
   Sparta, B., Hamilton, T., Hughes, S., Natesan, G., Deeds, E. J., 2023. A lack of distinct cell identities in single-cell measurements: revisiting Waddington's landscape. bioRxiv, 2022.06.03.494765.
   Waddington, C. H., 1957. The Strategy of the Genes; a Discussion of Some Aspects of Theoretical Biology. Allen & Unwin, London.
   Weinreb, C., Wolock,S., Tusi, B. K., Socolovsky, M., Klein, A. M., 2018. Fundamental limits on dynamic inference from single-cell snapshots. Proceedings of the National Academy of Sciences 115, E2467-E2476.
   Wu, F., Su, R. Q., Lai, Y. C., Wang, X., 2017. Engineering of a synthetic quadra stable gene network to approach Waddington landscape and cell fate determination. eLife 6, e23702.
   Zhou, J. X., Huang, S., 2011. Understanding gene circuits at cell-fate branch points for rational cell reprogramming. Trends Genet 27, 55-62.
   Zhou, J. X., Isik, Z., Xiao, C., Rubin, |., Kauffman, S. A., Schroeder, M., Huang, S., 2016. Systematic drug perturbations on cancer cells reveal diverse exit paths from proliferative state. Oncotarget 7, 7415-7425.

### Anhang

**Tabelle A**

| Tabelle A zeigt eine Liste von Genen der mittels des erfindungsgemäßen Verfahrens analysierten mRNAS | | | | | | |
|---|---|---|---|---|---|---|
| **Gen** | **Definition** | **Organismus** | **UniProt** | **Länge** | **E.Value** | **Identi** |
| *rps15A* | 40S ribosomales Protein S22-A cerevisiae (Stamm ATCC 204508 / | Saccharomyces cerevisiae (Stamm ATC | P0C0W1 | 130 | 7E-54 | 74 |
| *rps15A* | 40S ribosomales Protein S22-A cerevisiae (Stamm ATCC 204508 / | Saccharomyces cerevisiae (Stamm ATC | P0C0W1 | 130 | 7E-54 | 74 |
| *dspA* | ABC-Transporter F-Familienmitglied 4 discoldeum | Dictyostelium dlscoldeum | Q8T6B4 | 1142 | 4E-36 | 34 |
| *afk* | Aktin-Fragmin-Kinase Polycephalum | Physarum polycephalum | P80197 | 737 | 4E-16 | 23 |
| *afk* | Aktin-Fragmin-Kinase Polycephalum | Physarum polycephalum | P80197 | 737 | 4E-16 | 23 |
| *ardA* | Aktin, plasmodische Isoform polycephalum | Physarum polycephalum | P02576 | 376 | 2E-159 | 100 |
| *ardA* | Aktin, plasmodische Isoform polycephalum | Physarum polycephalum | P02576 | 376 | 8E-49 | 51 |
| *ak1* | Alpha-Protein-Kinase 1 discoideum | Dictyostelium discoideum | Q54DK4 | 1352 | 8E-15 | 25 |
| *vwkA* | Alpha-Protein-Kinase vwkA discoideum | Dictyostelium discoideum | Q6B9X6 | 625 | 4E-29 | 28 |
| *vwkB* | Alpha-Protein-Kinase vwkA discoideum | Dictyostelium discoideum | Q6B9X6 | 625 | 4E-15 | 31 |
| *anaA* | Anaphase-fördernder Komplex Untereinheit 1 musculus | Mus musculus | P53995 | 1944 | 0 | 30 |
| *apcA* | Anaphase-fördernder Komplex Untereinheit 11 thaliana | Arabidopsis thaliana | Q9M9L0 | 84 | 4E-32 | 60 |
| *fzrA* | Anaphasenfördernder Komplex Untereinheit cdc20 discoideum | Dictyostelium discoideum | Q54MZ3 | 499 | 1E-117 | 51 |
| *cdc20* | Untereinheit des Anaphase-fördernden Komplexes cdc20 discoide | Dictyostelium discoideum | Q54MZ3 | 499 | 2E-62 | 59 |
| *anxA* | Annexin A4 norvegicus | Rattus norvegicus | P55260 | 319 | 3E-30 | 35 |
| *pwiA* | argonaute-3 melanogaster_Protein ... | Drosophila melanogaster | Q7PLK0 | 867 | 3E-32 | 33 |
| *aurK* | Aurora-Kinase A-A laevis | Xenopus laevis | Q91820 | 407 | 5E-95 | 62 |
| *dim8* | Basischer Leuzin-Reißverschluss-Transkriptionsfaktor B discoideur | Dictyostelium discoideum | Q54ER9 | 602 | 6E-55 | 44 |
| *arpA* | Basic-Leucin-Zipper-Transkriptionsfaktor G discoideum _Wahrsch | Dictyostelium discoideum | Q54RZ9 | 372 | 2E-21 | 42 |
| *bzpJ* | Basis-Leucin-Zipper-TranskriptionsfaktorJ discoideum _Vermutllc | Dictyostelium discoideum | Q554P0 | 787 | 2E-24 | 26 |
| *bzpQ* | Basis-Leucin-Reißverschluss-Transkriptionsfaktor Q discoideum _\ | Dictyostelium discoideum | Q54IJ9 | 976 | 3E-65 | 40 |
| *cdcA* | Caltractin intestinalis | Giardia intestinalis | Q24956 | 176 | 4E-37 | 70 |
| *pkaR* | cAMP-abhängige Proteinkinase regulatorische Untereinheit einer | Blastocladiella emersonii | P31320 | 403 | 4E-24 | 32 |
| *cdc123* | Zellteilungszyklus-Protein 123 homolog sapiens | Homo sapiens | O75794 | 336 | 2E-10 | 30 |
| *ligA* | Kontrollpunkt-Protein hus1 homolog discoideum | Dictyostelium dlscoldeum | Q54NC0 | 271 | 9E-50 | 41 |
| *ralA* | Kreisförmig permutiertes Ras-Protein 1 discoideum | Dictyostelium discoideum | Q75J93 | 842 | 5E-21 | 41 |
| *cdc2* | Cyclin-abhängige Kinase 1 discoideum | Dictyostelium discoideum | P34112 | 296 | 9E-108 | 62 |
| *cdkA* | Cyclin-abhängige Kinase 2 sapiens | Homo sapiens | P24941 | 298 | 3E-117 | 69 |
| *cdkB1* | Cyclin-abhängige Kinase B1-2 thaliana | Arabidopsis thaliana | Q2V419 | 311 | 4E-14 | 28 |
| *cdkC* | Cyclin-abhängige Kinase C-2 sativa subsp. japonica | Oryza sativa subsp. japonica | Q5JK68 | 513 | 2E-131 | 57 |
| *cdkG2* | Cyclin-abhängige Kinase G-2 sativa subsp. japonica | Oryza sativa subsp. japonica | Q7XUF4 | 710 | 3E-102 | 54 |

| **Gen** | **Definition** | **Organismus** | **UniProt** | **Länge** | **E.Value** | **Identl** |
|---|---|---|---|---|---|---|
| *cdk11* | Cyclin-abhängige Kinase G-2 sativa subsp. japonica | Oryza sativa subsp. japonica | Q7XUF4 | 710 | 3E-126 | 56 |
| *cycA* | Cyclin-H1-1 sativa subsp. japonica | Oryza sativa subsp. japonica | Q10D80 | 330 | 1E-42 | 33 |
| *ccnA* | Cyclin-L1-1 sativa subsp. japonica | Oryza sativa subsp. japonica | Q9AS36 | 427 | 3E-75 | 37 |
| *cycU2* | Cyclin-P3-1 sativa subsp. japonica | Oryza sativa subsp. japonica | Q75HV0 | 236 | 1E-24 | 38 |
| *cycT* | Zyklin-T1-5 thaliana | Arabidopsis thaliana | Q9FKE6 | 579 | 8E-60 | 44 |
| *ccnQ* | Zyklin-T1-5 thaliana | Arabidopsis thaliana | Q9FKE6 | 579 | 1E-50 | 38 |
| *cycU4A* | Cyclin-U4-2 thaliana | Arabidopsis thaliana | Q9LY16 | 216 | 5E-19 | 29 |
| *cycU48* | Zyklin-U4-3 thaliana | Arabidopsis thaliana | Q9FKF6 | 219 | 3E-19 | 31 |
| *ccnY2* | Cyclin-Y musculus | Mus musculus | Q8BGU5 | 341 | 2E-34 | 36 |
| *ccnY1* | Cyclin-Y sapiens | Homo sapiens | Q8ND76 | 341 | 3E-50 | 41 |
| *damA* | DNA-Schadensbindungsprotein 1 sativa subsp. japonica | Oryza sativa subsp. japonica | Q6L4S0 | 1090 | 0 | 61 |
| *mutL* | DNA-Mismatch-Reparatur-Protein Mlh3 sapiens | Homo sapiens | Q9UHC1 | 1453 | 2E-43 | 33 |
| *mlh3* | DNA-Fehlanpassungs-Reparaturprotein MLH3 thaliana | Arabidopsis thaliana | F4JN26 | 1155 | 9E-30 | 27 |
| *msh5* | DNA-Fehlpaarungs-Reparaturprotein MSH5 thaliana | Arabidopsis thaliana | F4JEP5 | 807 | 8E-128 | 35 |
| *rpb2* | DNA-gesteuerte RNA-Polymerase II-Untereinheit rpb2 discoideum | Dictyostelium discoideum | Q54J75 | 1170 | 0 | 74 |
| *ehdA* | EH-Domäne-enthaltendes Protein 1 abelii | Pongo abelii | Q5RBP4 | 534 | 6E-110 | 40 |
| *erkA* | Extrazellulärsignal-regulierte Kinase 1 discoideum | Dictyostelium discoideum | P42525 | 529 | 1E-141 | 67 |
| *erkb* | Extrazellulärsignal-regulierte Kinase 2 discoideum | Dictyostelium discoideum | Q54QB1 | 369 | 1E-171 | 82 |
| *cycB2* | G1/S-spezifisches Cyclin-E pulcherrimus | Hemicentrotus pulcherrimus | 015995 | 424 | 3E-28 | 27 |
| *cycB* | G2/mitose-spezifisches Cyclin-B discoideum | Dictyostelium discoideum | P42524 | 436 | 7E-85 | 42 |
| *gtaL* | GATA-Zinkfingerdomäne-enthaltendes Protein 12 discoideum | Dictyostelium discoideum | Q54NM5 | 640 | 1E-24 | 27 |
| *ml2* | Glycin-reiches Zellwand-Strukturprotein thaliana | Arabidopsis thaliana | P27483 | 349 | 1E-60 | 45 |
| *rasA* | GTP-bindendes Protein YPTM2 mays | Zea mays | Q05737 | 203 | 2E-35 | 38 |
| *hcpA* | Histon-Chaperon ASF1A taurus | Bos taurus | Q2KIG1 | 204 | 4E-51 | 52 |
| *hstA* | Histon H2B-Nekatrix | Rosellinia necatrix | Q8J1K2 | 136 | 4E-36 | 51 |
| *hstA* | Histon H2B-Nekatrix | Rosellinia necatrix | Q8J1K2 | 136 | 4E-36 | 51 |
| *hbx2* | Homeobox-Protein 2 discoideum | Dictyostelium discoideum | Q869W0 | 942 | 2E-14 | 37 |
| *dhkl1* | Hybride Signaltransduktion Histidinkinase I discoideum | Dictyostelium discoideum | Q86AT9 | 1736 | 2E-23 | 38 |
| *dhkH* | Hybride Signaltransduktion Histidinkinase J discoideum | Dictyostelium discoideum | Q54YZ9 | 2062 | 7E-57 | 43 |
| *dhkJ* | Hybride Signalübertragungs-Histidinkinase J discoideum | Dictyostelium discoideum | Q54YZ9 | 2062 | 4E-57 | 44 |
| *pptA* | inaktive violette saure Phosphatase 29 thaliana _Wahrscheinlich | .Arabidopsis thaliana | Q9FMK9 | 389 | 4E-09 | 46 |
| *det1* | Licht-vermitteltes Entwicklungsprotein DET1 thaliana | Arabidopsis thaliana | P48732 | 543 | 6E-83 | 32 |
| *cdc25* | M-Phaseninduktor-Phosphatase 3 taurus | Bos taurus | A5D7P0 | 477 | 4E-32 | 33 |
| *mpl2* | MAP-Kinase-Phosphatase mit leucinreichen Wiederholungen Protein | Dictyostelium discoideum | Q54Y32 | 856 | 1E-24 | 45 |
| *meiB* | MEI2-wie 5 thaliana _Protein ... | Arabidopsis thaliana | Q8VWF5 | 800 | 2E-78 | 64 |
| *mei8* | MEI2-wie 5 thaliana _Protein ... | Arabidopsis thaliana | Q8VWF5 | 800 | 2E-78 | 64 |
| *mkkA* | Mitogen-aktivierte Proteinkinase Kinase Kinase A discoideum | Dictyostelium discoideum | Q54R82 | 942 | 4E-96 | 59 |
| *bub2* | Mitotischer Kontrollpunkt Protein BUB2 discoideum _Putativ... | Dictyostelium discoideum | Q55EP9 | 366 | 2E-93 | 54 |
| *bub1* | Mitotischer Kontrollpunkt Serin/Threonin-Protein-Kinase BUB1 th | Arabidopsis thaliana | F4IVI0 | 525 | 2E-59 | 40 |
| *modL* | Mitotischer Spindelaufbau-Kontrollpunkt-Protein MAD1 sapiens | Homo sapiens | Q9Y6D9 | 718 | 3E-22 | 38 |
| *mod2L1* | Mitotischer Spindelaufbau-Checkpoint-Protein MAD2A discoideum | Dictyostelium discoideum | Q556Y9 | 203 | 8E-68 | 64 |
| *more* | Mmusculus | Mus musculus | F7BJB9 | 942 | 2E-102 | 52 |
| *msh4* | MutS-Protein-Homolog 4 musculus | Mus musculus | Q99MT2 | 958 | 3E-136 | 35 |
| *mybF* | Myb-ähnliches Protein D discoideum | Dictyostelium discoideum | Q54K19 | 595 | 4E-21 | 41 |
| *mybD* | Myb-ähnliches Eiweiß D discoideum | Dictyostelium discoideum | Q54K19 | 595 | 1E-20 | 41 |
| *secG* | Myb-ähnliches Protein X discoideum | Dictyostelium discoideum | Q54J55 | 1620 | 2E-21 | 26 |
| *aroL* | NADH-Ubichinon-Oxidoreduktase-Kette 3 citrinum | Dictyostelium citrinum | Q2LCR0 | 120 | 0.23 | 28 |
| *aroL* | NADH-Ubichinon-Oxidoreduktase-Kette 3 citrinum | Dictyostelium citrinum | Q2LCR0 | 120 | 0.23 | 28 |
| *znfX1* | Zinkfinger-enthaltendes Protein vom NFX1-Typ 1 musculus | Mus musculus | Q8R151 | 1909 | 0 | 31 |
| *nhpA* | Chromosomales Nicht-Histon-Protein 6 maydis (Stamm 521 / FGS | Ustilago maydis (strain 521 / FGSC 902 | Q4PBZ9 | 99 | 4E-17 | 48 |
| *nhpA* | Nicht-Histon-Chromosomenprotein 6 maydis (Stamm 521 / FGSC | Ustilago maydis (strain 521 / FGSC 902 | Q4PBZ9 | 99 | 4E-17 | 48 |
| *pikB* | Phosphatidylinositol-3-Kinase 2 discoideum | Dictyostelium discoideum | P54674 | 1857 | 0 | 55 |
| *pikC* | Phosphatidylinositol-4-Kinase beta musculus | Mus musculus | Q8BKC8 | 816 | 6E-70 | 32 |
| *pldA* | Phosphatidylinositol-Glykan-spezifische Phospholipase D norvegic | Rattus norvegicus | Q8R2H5 | 843 | 4E-70 | 27 |
| *pldB* | Phosphatidylinositol-Glykan-spezifische Phospholipase D sapiens | Homo sapiens | P80108 | 840 | 5E-51 | 27 |
| *pldC* | Phospholipase D A discoideum | Dictyostelium discoideum | Q54UK0 | 1269 | 3E-53 | 31 |
| *ribA* | Poly(ADP-ribose)-Glykohydrolase melanogaster | Drosophila melanogaster | O46043 | 723 | 7E-89 | 40 |
| *ribB* | Prä-mRNA-verarbeitender Faktor 19 sativa subsp. japonica | Oryza sativa subsp. japonica | Q9AV81 | 527 | 4E-123 | 50 |
| *ribB* | Prä-mRNA-verarbeitender Faktor 19 sativa subsp. japonica | Oryza sativa subsp. japonica | Q9AV81 | 527 | 4E-123 | 50 |
| *ribB* | Prä-mRNA-verarbeitender Faktor 19 sativa subsp. japonica | Oryza sativa subsp. japonica | Q9AV81 | 527 | 4E-123 | 50 |
| *pcnA* | Proliferierendes Zellkern-Antigen napus | Brassica napus | Q43124 | 263 | 1E-78 | 55 |
| *pcnA* | Proliferierendes Zellkern-Antigen napus | Brassica napus | Q43124 | 263 | 1E-78 | 55 |

| **Gen** | **Definition** | **Organismus** | **UnIProt** | **Länge** | **E.Value** | **Identi** |
|---|---|---|---|---|---|---|
| *pptB* | Proteinphosphatase 2C 43 sativa subsp. japonica _Wahrscheinlich | Oryza sativa subsp. japonica | Q7XUC5 | 388 | 1E-24 | 31 |
| *pumA* | Pumilio homolog 1 thaliana | Arabidopsis thaliana | Q9ZW07 | 968 | 3E-133 | 36 |
| *rgsA* | Regulator der G-Protein-Signalübertragung 2 musculus | Mus musculus | O08849 | 211 | 0.014 | 41 |
| *spiA* | Rootletin musculus | Mus musculus | Q8CJ40 | 2009 | 0.0002 | 25 |
| *scaper* | S-Phasen-Cyclin A-assozilertes Protein im endoplasmatischen Reti | Homo sapiens | Q9BY12 | 1400 | 1E-57 | 25 |
| *uchA* | Sekretorisches Immunglobulin A-bindendes Protein EsiB coli | Escherichia coli O6:H1 (strain CFT073 / | A0A0H2VD | 490 | 2E-15 | 33 |
| *atm* | Serin-Protein-Kinase ATM musculus | Mus musculus | Q62388 | 3066 | 0 | 29 |
| *rps6KA5* | Serin/Threonin-Proteinkinase fhkE discoideum _Wahrscheinlich .. | Dictyostelium discoideum | Q54VI1 | 712 | 3E-76 | 32 |
| *mrkC* | Serin/Threonin-Protein-Kinase MARK-B discoideum _Wahrscheinl | Dictyostelium discoideum | Q54MV2 | 715 | 6E-109 | 35 |
| *pakA* | Serin/Threonin-Protein-Kinase pakA discoideum | Dictyostelium discoideum | Q55D99 | 1197 | 3E-84 | 42 |
| *roco1* | Serin/Threonin-Protein-Kinase pats1 discoideum _Wahrscheinlich | Dictyostelium discoideum | Q55E58 | 3184 | 1E-10 | 22 |
| *pksA* | Serin/Threonin-Protein-Kinase phg2 discoideum | Dictyostelium discoideum | Q54QQ1 | 1387 | 3E-87 | 37 |
| *skp1* | SKP1-ähnliches Protein 21 thaliana | Arabidopsis thaliana | Q8LF97 | 351 | 3E-11 | 39 |
| *spdYC* | Speedy-Protein A norvegicus | Rattus norvegicus | Q8R496 | 312 | 3E-06 | 26 |
| *spaTA5L1* | Spermatogenese-assoziiertes Protein 5-ähnliches Protein 1 sapier | Homo sapiens | Q9BVQ7 | 753 | 8E-133 | 40 |
| *tspA* | Sporenwandprotein 2 intestinalis | Encephalitozoon intestinalis | Q95WA4 | 1002 | 2E-27 | 24 |
| *cklA* | T-Zell-Leukämie-Homeobox-Protein 3 gallus | Gallus gallus | O93367 | 297 | 5E-06 | 40 |
| *hcaD* | Hodenexprimiertes Protein 30 musculus | Mus musculus | Q3TUU5 | 225 | 3E-09 | 33 |
| *agdA* | Transkriptionsfaktor SPT20 homolog discoideum | Dictyostelium discoideum | Q54VY3 | 1402 | 1E-34 | 39 |
| *pbrM1* | Transkriptions-Initiationsfaktor TFIID Untereinheit 1 discoideum | Dictyostelium discoideum | Q54DH8 | 2310 | 7E-27 | 24 |
| *mybQ* | Transkriptionsaktivator Myb gallus | Gallus gallus | P01103 | 641 | 3E-58 | 59 |
| *cudB* | Transkriptionsregulator cudA discoideum _Putativ ... | Dictyostelium discoideum | 000841 | 802 | 5E-64 | 60 |
| *altA* | Tubulin alpha-1A-Kette polycephalum | Physarum polycephalum | P50258 | 450 | 1E-136 | 77 |
| *gapA* | Nicht charakterisiertes Protein C622.14 pombe (Stamm 972 / ATC | Schizosaccharomyces pombe (strain 97 | O94601 | 321 | 8E-21 | 37 |
| *cudA* | Nicht charakterisiertes Protein DDB_G0286447 discoideum | Dictyostelium discoideum | Q54LR3 | 521 | 2E-50 | 50 |
| *psgA* | Nicht charakterisiertes Transmembranprotein DDB_G0284159 dis | Dictyostelium discoideum | Q54Q42 | 86 | 0.069 | 100 |
| *wee1* | Wee1-ähnliche Proteinkinase musculus | Mus musculus | P47810 | 646 | 8E-57 | 39 |
| *ncbP* | Zinkfinger-CCCH-Domäne-enthaltendes Protein 13 musculus | Mus musculus | E9Q784 | 1729 | 2E-34 | 36 |
| *znfA* | Zinkfinger-Protein 732 sapiens | Homo sapiens | B4DXR9 | 585 | 5E-14 | 26 |
| | | | | | | |

### Bezugszeichenliste

- On: ansteigen
- Off: abfallen
- Lo: niedrig
- In: mittel bzw. inaktiv
- Hi: hoch

## Patentansprüche

1. Verfahren zur Analyse von Schalt- und Regulationsprozessen, welches folgende Schritte umfasst:
a) Bereitstellen mindestens eines Reaktionssystems,
b) Messen und auswählen mindestens eines Parameters des mindestens einen Reaktionssystems aus Schritt a),
c) Mindestens eine Wiederholung von Schritt b),
d) Erstellen mindestens einer Zeitserie mittels mindestens eines in Schritt b) ausgewählten Parameters,
e) Diskretisieren der gemessenen Parameterwerte aus Schritt b) in der Zeitserie aus Schritt d) zur Erzeugung einer Abfolge von Mikrozuständen,
f) Erzeugen eines gerichteten Graphen über Makrozustände aus der mindestens einen diskretisierten Zeitserie in Schritt e),
g) Strukturelle Analyse des gerichteten Graphen aus Schritt f) zur kombinatorischen Ermittlung der Zahl der zeitlich korrelierten Änderungen der Mikrozustände in einer Teilmenge zufällig ausgewählter Systemkomponenten.

2. Verfahren, gemäß Anspruch 1, **dadurch gekennzeichnet, dass** das mindestens eine Reaktionssystem in einer eukaryotischen oder prokaryotischen Zellen enthalten ist oder aus solchen Zellen besteht.

3. Verfahren, gemäß Anspruch 1 oder 2, **dadurch gekennzeichnet, dass** nach Schritt a) ein Schritt a1) erfolgt, in dem an dem mindestens einen Reaktionssystem eine Pertubation erfolgt.

4. Verfahren, gemäß Anspruch 3, **dadurch gekennzeichnet, dass** die Pertubation als Induktion einer Zelle durch Stimulation erfolgt.

5. Verfahren, gemäß Anspruch 1 oder 2, **dadurch gekennzeichnet, dass** in Schritt d) ein Startpunkt aus den Messdaten in Schritt b) für die Erstellung der Zeitserie bestimmt wird.

6. Verfahren, gemäß mindestens einem der vorangehenden Ansprüche, **dadurch gekennzeichnet, dass** in Schritt b) der mindestens eine Parameter die zelluläre Konzentration von Biomolekülen, von deren kovalenten Modifikationen oder die Konzentration vorhandener Komplexe (Aggregate) von Biomolekülen ist.

7. Verfahren, gemäß Anspruch 6, **dadurch gekennzeichnet, dass** ein Logarithmus der Konzentration jedes Parameters gegen ein definiertes zeitliches Intervall aufgetragen und graphisch dargestellt wird.

8. Verfahren, gemäß mindestens einem der vorangehenden Ansprüche, **dadurch gekennzeichnet, dass** die mindestens eine Wiederholung in Schritt c) in einem zeitlich definierten Intervall erfolgt.

9. Verfahren, gemäß mindestens einem der vorangehenden Ansprüche, **dadurch gekennzeichnet, dass** die Makrozustände durch die Änderung mindestens eines Parameters der Mikrozustände in Schritt e) hervorgerufen werden.

10. Verfahren, gemäß Anspruch 9, **dadurch gekennzeichnet, dass** die Änderung ein Anstieg oder ein Abfall oder eine Oszillation eines Parameters oder keine Veränderung ist.

11. Verfahren, gemäß mindestens einem der vorangehenden Ansprüche, **dadurch gekennzeichnet, dass** die Abfolge von Makrozuständen in Form eines Graphen dargestellt wird, wobei der gerichtete Graph ein endlicher Automat ist.

12. Verfahren, gemäß Anspruch 11, **dadurch gekennzeichnet, dass** der endliche Automat als ein Petrinetz dargestellt ist.

13. Verfahren, gemäß mindestens einem der vorangehenden Ansprüche, **dadurch gekennzeichnet, dass** in Schritt g) unterschiedliche Makrozustände bestimmt werden, die in einem gemeinsamen Makrozustand enden.

14. Verfahren, gemäß Anspruch 13, **dadurch gekennzeichnet, dass** in Schritt g) die Anzahl gleicher Makrozustandsänderungen bestimmt wird, die in einem gemeinsamen Makrozustand enden.

15. Verfahren, gemäß Anspruch 13, **dadurch gekennzeichnet, dass** eine Änderung im Mikrozustand eines Parameters identifiziert wird, der vor dem Hintergrund anderer Parameter auftritt, deren Mikrozustand sich zu dem jeweiligen Zeitpunkt noch nicht oder nicht mehr ändert.

16. Verfahren, gemäß mindestens einem der vorangehenden Ansprüche, **dadurch gekennzeichnet, dass** in Schritt g) alle Anfangs- und Endmakrozustände und deren Anzahl bestimmt wird.
